# FASCICULE DE BREVET EUROPEEN

(11) **EP 1 274 350 B1**
(45) Date de publication et mention de la délivrance du brevet: **12.09.2007**
(21) Numéro de dépôt: 01919593.2
(22) Date de dépôt: 29.03.2001
(51) Int. Cl.: A61B 17/04

(54) **APPLICATEUR POUR LA MISE EN PLACE D'UNE AIGUILLE CHIRURGICALE**
APPLIKATOR ZUM EINBRINGEN EINER CHIRURGISCHEN NADEL
MANIPULATOR FOR FIXING A SURGICAL NEEDLE

(30) Priorité: 21.04.2000 FR 0005127
(43) Date de publication de la demande: 15.01.2003
(73) Titulaire: SOPRANE SA, F-69006 Lyon (FR)
(72) Inventeur: LOUBENS, Thierry, F-69370 Saint Didier au Mont d'Or (FR); RIOU, Lionel, F-69003 Lyon (FR); WATRELOT, Antoine, F-69008 Lyon (FR)
(74) Mandataire: Schmitt, John
(86) Numéro de dépôt international: PCT/FR2001/000948
(87) Numéro de publication internationale: WO 2001/080747

(56) Documents cités:
- EP-A- 0 599 772
- WO-A-92/05828
- WO-A-93/13714
- WO-A-98/57584
- US-A- 5 391 174

## Description

La présente invention est relative à un applicateur pour la mise en place d'une aiguille chirurgicale solidaire de son fil de suture dans le site opératoire, et plus particulièrement à des perfectionnements permettant d'indexer angulairement la position de l'aiguille chirurgicale par rapport audit applicateur.

L'applicateur suivant la présente invention est destiné aux procédures endoscopiques, afin de coopérer, par exemple, avec un trocart de faible diamètre pour amener et libérer l'aiguille chirurgicale dans le site opératoire.

On connaît d'après la demande de brevet EP 0 554 361 un applicateur comportant une canule cylindrique solidaire à l'une de ses extrémités de poignées pour son maintien par le chirurgien, d'un manchon creux retenant à l'une de ses extrémités l'aiguille chirurgicale, tandis que l'extrémité opposée est solidaire d'une poignée pour permettre le coulissement du manchon par le chirurgien à l'intérieur de la canule, afin de libérer l'aiguille dans le site opératoire.

L'extrémité libre du manchon creux retenant l'aiguille chirurgicale est réalisée sous la forme d'un mandrin comportant des mors de serrage qui se déforment axialement du fait de la différence de diamètre entre celui interne de la canule et celui externe du mandrin pour que lesdits mors viennent se serrer autour de l'aiguille et la retenir à l'intérieur de la canule.

L'extraction du mandrin à l'extérieur de la canule par coulissement du manchon à l'intérieur de cette dernière, permet de libérer la pression de serrage sur les mors afin que le chirurgien puisse à l'aide d'une pince, récupérer l'aiguille chirurgicale maintenue en position dans ledit mandrin.

On note que le dispositif de retenue de l'aiguille chirurgicale dans l'applicateur permet de maintenir cette dernière dans une position angulaire déterminée par rapport au manchon creux, mais pas par rapport à la canule, car ledit manchon peut tourner à l'intérieur de ladite canule.

La rotation du manchon à l'intérieur de la canule entraîne que l'aiguille sorte dans une position qui est aléatoire et toujours différente par rapport à la canule, obligeant le chirurgien à repositionner l'aiguille dans le site opératoire pour pouvoir effectuer le premier point de suture.

On connaît également d'après le brevet FR 97 07681 appartenant au demandeur, un applicateur pour la mise en place d'une aiguille chirurgicale dans le site opératoire

L'applicateur décrit dans le brevet FR 97 07681 comprend un manchon dans lequel est contraint dans une position sensiblement allongée, une aiguille, une tige qui pénètre dans le manchon pour l'extraction de ladite aiguille, et des moyens de retenue qui permettent, avant l'extraction de l'aiguille, de maintenir la tige à l'intérieur du manchon dans une position déterminée de manière que l'extrémité libre de ladite tige soit en appui contre l'aiguille.

En outre, l'applicateur comporte dans la partie interne du manchon des moyens de tension qui permettent, pendant l'extraction de l'aiguille, de maintenir le fil de suture tendu.

L'applicateur suivant la présente invention consiste à remédier aux inconvénients de l'applicateur décrit dans la demande de brevet EP 0 554 361 en permettant au chirurgien de savoir dans quelle position l'aiguille chirurgicale va sortir dans le site opératoire.

L'applicateur suivant la présente invention à pour objet de perfectionner l'applicateur décrit dans le brevet FR 97 07681, afin de permettre, d'une part à l'aiguille chirurgicale de sortir dans une position déterminée et constante par rapport au manchon, et d'autre part à limiter la course de la tige d'extraction de l'aiguille par rapport au manchon.

L'applicateur pour la mise en place d'une aiguille chirurgicale solidaire d'un fil de suture suivant la présente invention comprend un manchon dans lequel est contraint dans une position sensiblement allongée l'aiguille chirurgicale, une tige qui pénètre dans le manchon pour l'extraction de ladite aiguille et des moyens d'indexation angulaire qui consistent en une conformation du manchon au niveau de l'une de ses extrémités libres de manière que l'alésage interne dudit manchon présente un profil interne oblong dont les dimensions sont différentes pour permettre lors de l'extraction de l'aiguille chirurgicale du manchon de la diriger dans une position déterminée et constante par rapport aux axes principaux dudit manchon.

L'applicateur pour la mise en place d'une aiguille chirurgicale solidaire d'un fil de suture suivant la présente invention comprend des moyens d'indexation angulaire qui consistent en une conformation du diamètre externe D1 du manchon, afin que l'alésage interne dudit manchon présente ledit profil interne oblong dont la largeur (e) est inférieure au diamètre externe D1, tandis que sa hauteur H est semblable à celle du diamètre externe D1 pour permettre, lors de l'extraction de l'aiguille chirurgicale du manchon, de la diriger dans une position déterminée et constante par rapport aux axes principaux dudit manchon.

L'applicateur pour la mise en place d'une aiguille chirurgicale solidaire d'un fil de suture suivant la présente invention comprend un diamètre extérieur D1 du manchon qui est déformé latéralement pour que l'extrémité présente un profil qui est constitué de deux méplats parallèles se raccordant au profil cylindrique du manchon par des portions inclinées.

L'applicateur pour la mise en place d'une aiguille chirurgicale solidaire d'un fil de suture suivant la présente invention comprend un alésage interne qui présente entre son profil interne oblong et son diamètre circulaire interne, une portion inclinée provenant des portions inclinées extérieures.

L'applicateur pour la mise en place d'une aiguille chirurgicale solidaire d'un fil de suture suivant la présente invention comprend un profil oblong interne qui se termine par une chanfrein interne permettant de positionner et de faciliter l'introduction de l'aiguille à l'intérieur de l'alésage interne du manchon lorsqu'il est nécessaire de retirer ladite aiguille du site opératoire.

L'applicateur pour la mise en place d'une aiguille chirurgicale solidaire d'un fil de suture suivant la présente invention comprend un profil interne oblong communiquant avec l'alésage interne du manchon et qui présente au niveau du chanfrein interne un profil circulaire de diamètre externe D2 qui est inférieur à celui D1 dudit manchon donnant à l'extrémité libre une forme sensiblement de trompette.

L'applicateur pour la mise en place d'une aiguille chirurgicale solidaire d'un fil de suture suivant la présente invention comprend un manchon dont le profil du diamètre interne est obtenue par conformation d'un tube métallique.

L'applicateur pour la mise en place d'une aiguille chirurgicale solidaire d'un fil de suture suivant la présente invention comprend un manchon dont le profil du diamètre interne est obtenue lors du moulage dudit manchon.

L'applicateur pour la mise en place d'une aiguille chirurgicale solidaire d'un fil de suture suivant la présente invention comprend une tige qui comporte une tête solidaire de languettes souples permettant de limiter la course de la tige par rapport au manchon.

L'applicateur pour la mise en place d'une aiguille chirurgicale solidaire d'un fil de suture suivant la présente invention comprend des moyens de retenue qui sont constitués d'une platine de forme oblongue, dont l'une des faces est solidaire de deux pinces qui coopèrent respectivement avec la tige et le manchon.

L'applicateur pour la mise en place d'une aiguille chirurgicale solidaire d'un fil de suture suivant la présente invention comprend un manchon qui comporte une tête cylindrique pourvue sur sa face externe d'une empreinte circulaire qui coopère avec la pince de la platine, tandis que la tige comprend une tête munie d'une empreinte autour de laquelle s'encliquette la pince de ladite platine de manière à maintenir une distance L constante entre les têtes pour assurer que l'extrémité libre de la tige soit, d'une part en contact avec l'aiguille, et d'autre part à une distance L' de l'extrémité libre du manchon.

L'applicateur pour la mise en place d'une aiguille chirurgicale solidaire d'un fil de suture suivant la présente invention comprend un manchon qui comporte à partir de l'extrémité, et en direction de la tête, deux rainures diamétralement opposées et parallèles à l'axe longitudinal de l'applicateur.

L'applicateur pour la mise en place d'une aiguille chirurgicale solidaire d'un fil de suture suivant la présente invention comprend des rainures qui présentent un profil en forme de V dont la base la plus ouverte, ou la plus large, est tournée du côté de l'extrémité.

L'applicateur pour la mise en place d'une aiguille chirurgicale solidaire d'un fil de suture suivant la présente invention comprend des rainures qui présentent une profondeur décroissante le long de la périphérie du manchon, afin que le niveau le plus profond soit du côté de l'extrémité libre et de la base la plus ouverte du V.

L'applicateur pour la mise en place d'une aiguille chirurgicale solidaire d'un fil de suture suivant la présente invention comprend une extrémité du manchon qui comporte une fente disposée perpendiculairement aux rainures de manière que chaque rainure coopère avec ladite fente.

L'applicateur pour la mise en place d'une aiguille chirurgicale solidaire d'un fil de suture suivant la présente invention comprend une fente qui forme une creusure à l'intérieur du manchon qui communique avec l'alésage interne.

La description qui va suivre en regard des dessins annexés, donnés à titre d'exemples non limitatifs, permettra de mieux comprendre l'invention, les caractéristiques qu'elle présente et les avantages qu'elle est susceptible de procurer :
Figure 1 est une coupe illustrant l'applicateur pour la mise en place d'une aiguille suivant la présente invention.
Figures 2 à 4 sont des vues représentant les moyens d'indexation angulaire pour le positionnement de l'aiguille chirurgicale lors de l'extraction du manchon de l'applicateur suivant la présente invention.
Figures 5 à 7 sont des vues montrant une variante des moyens d'indexation angulaire représenté en figures 2 à 4.
Figure 8 est une vue illustrant en détail le profil en forme de crochet de la tige constituant le pousse aiguille de l'applicateur suivant la présente invention.
Figures 9 et 10 sont des vues montrant l'extrémité libre du manchon obtenue directement par moulage.
Figures 11 a à 11 h sont des vues représentant les différentes étapes de mise en place et d'utilisation de l'applicateur suivant la présente invention.

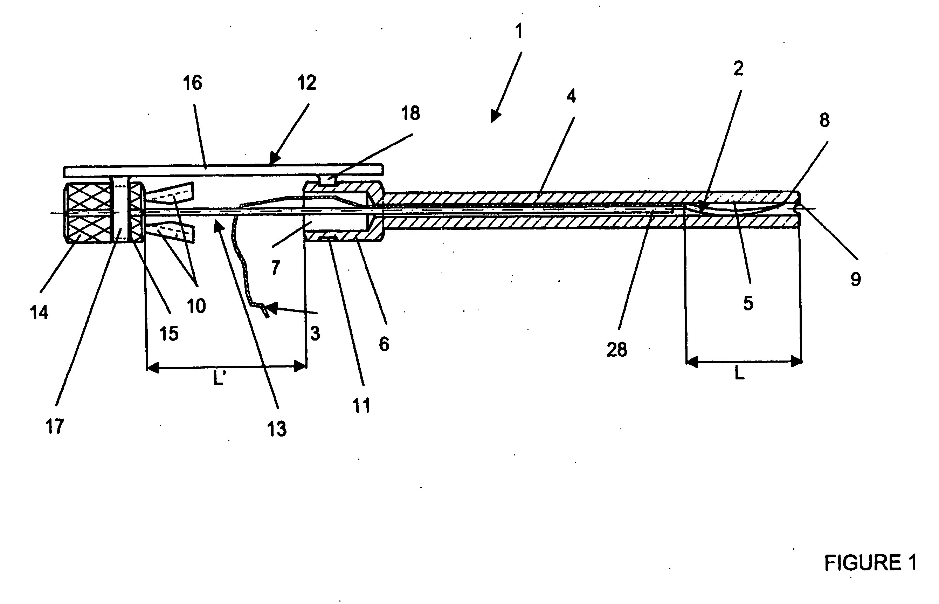

On a représenté en figure 1 un applicateur 1, semblable à celui décrit dans le brevet FR 97 07681, permettant la mise en place d'une aiguille chirurgicale 2 solidaire d'un fil de suture 3 dans le site chirurgical par l'intermédiaire d'un trocart, non représenté, et qui a été préalablement placé au travers de la paroi d'un patient.

L'applicateur 1 comprend un manchon cylindrique 4 percé sur toute sa longueur, et parallèlement à son axe longitudinal, d'un alésage interne 5 dans lequel sont logés l'aiguille 2 et son fil de suture 3.

Le manchon 4 comporte à l'une de ses extrémités une tête cylindrique 6 qui est prévue d'un diamètre supérieur à celui du reste du corps dudit manchon. A l'intérieur de la tête 6 est percé, coaxialement à l'alésage 5, un logement cylindrique 7 qui est d'un diamètre supérieur à celui dudit alésage.

Le logement 7 communique avec l'alésage interne 5 de manière que le fil de suture 3 solidaire de l'aiguille 2, puisse sortir en direction de l'extérieur du manchon 4.

En effet, l'aiguille 2 est disposée dans l'alésage interne 5 du manchon 4 de manière que sa pointe 8 soit dirigée en direction de l'extrémité libre 9 et opposée à celle portant la tête 6.

La paroi externe de la tête 6 présente une empreinte circulaire 11 coopérant avec des moyens de retenue 12 qui consistent à maintenir dans une position déterminée une tige 13 ou pousse aiguille, par rapport au manchon 4.

La tige 13 est solidaire à l'une de ses extrémités d'une tête 14 comportant sur sa face externe une empreinte circulaire 15 prévue pour coopérer avec les moyens de retenue 12.

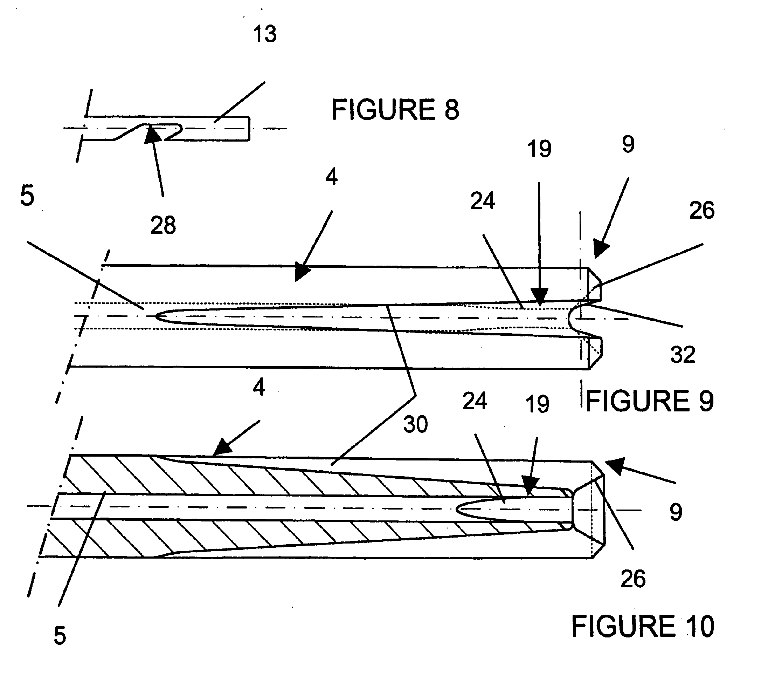

La tige 13 comporte au niveau de son extrémité libre opposée à la tête 14 un crochet 28 destiné à récupérer le fil de suture 3 de l'aiguille 2 (figure 8).

La tête 14 comprend dans son prolongement et de part et d'autre de la tige 13, des languettes souples 10 en forme de pinces constituant à l'état normal une butée pour limiter la course de déplacement de la tige 13 par rapport au manchon 4.

La tige 13 est introduite dans le manchon 4 de manière à venir en appui contre l'aiguille 2. La tige 13 traverse le logement 7 et l'alésage interne 5, de manière que le fil de suture 3 soit disposé entre ladite tige et la paroi dudit alésage.

Les moyens de retenue 12 permettent de maintenir la tige 13 par rapport au manchon 4, de manière que l'extrémité libre de la tige 13 opposée à la tête 14 soit toujours en appuie contre l'aiguille 2.

Les moyens de retenue 12 sont constitués d'une platine 16 solidaire sur l'une de ses faces de deux pinces 17 et 18 formant deux clips de fixation. Chaque pince 17 et 18 coopère respectivement avec l'empreinte 15 de la tête 14 solidaire de la tige 13, et l'empreinte 11 de la tête 6 du manchon 4.

La position des deux pinces 17 et 18 sur la platine 16 permet de maintenir entre la tête 14 de la tige 13 et la tête 6 du manchon 4 une distance constante L.

Cette distance L assure que l'extrémité libre de la tige 13, c'est à dire celle opposée à la tête 14, soit, d'une part en contact avec l'aiguille 2, et d'autre part à une distance L' identique à celle L de l'extrémité libre 9 du manchon 4.

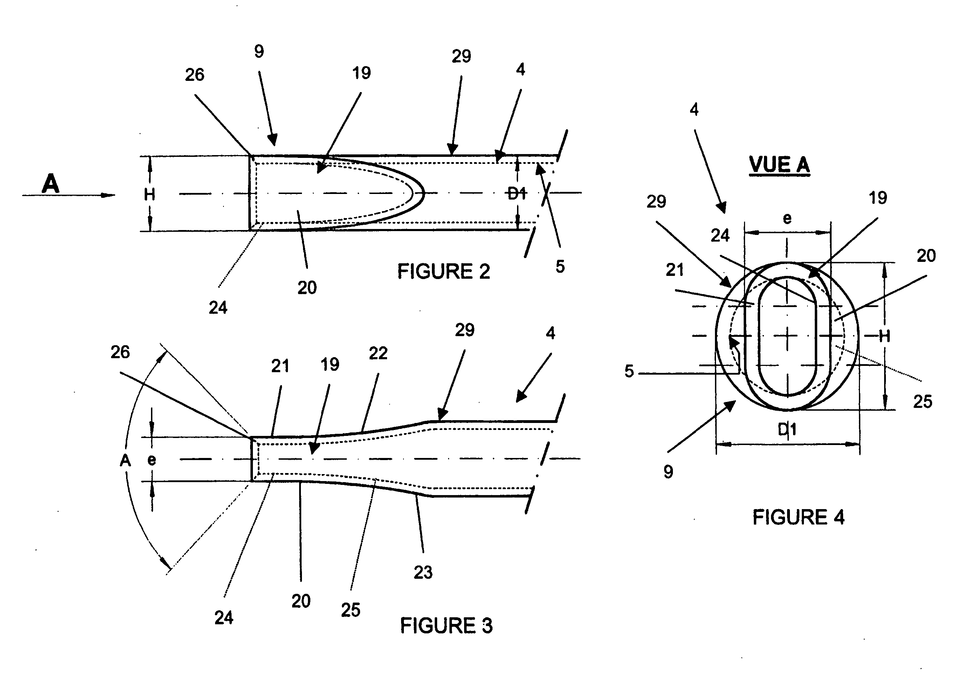

En figures 2 à 4 on a montré le profil de l'extrémité libre 9 du manchon 4 lorsque ce dernier est constitué d'un tube métallique 29 autour duquel est surmoulé une enveloppe plastique cylindrique non représentée.

L'extrémité libre 9 comporte des moyens d'indexation angulaire 19 de l'aiguille chirurgicale 2 par rapport aux axes principaux dudit manchon.

Les moyens d'indexation angulaire 19 consistent en une conformation du diamètre externe D1 du manchon 4 afin que l'alésage interne 5 dudit manchon présente un profil particulier qui permet lors de l'extraction de l'aiguille chirurgicale 2 de la diriger dans une position déterminée et constante par rapport aux axes principaux du manchon 4.

Le diamètre extérieur D1 du manchon 4 est déformé latéralement pour que l'extrémité 9 présente un profil qui est constitué de deux méplats parallèles 20 et 21 se raccordant au profil cylindrique du manchon par des portions inclinées 22 et 23.

La déformation du diamètre D1 du manchon 4 conduit à la déformation de l'alésage interne 5 qui présente un profil interne oblong 24 dont la largeur e est inférieure au diamètre externe D1, tandis que sa hauteur H est semblable à celle du diamètre externe D1.

L'alésage interne 5 présente entre son profil interne oblong 24 et son diamètre circulaire interne, une portion inclinée 25 provenant des portions inclinées extérieures 22 et 23.

La portion inclinée interne 25 permet de diriger l'aiguille 2 dans le sens du profil interne oblong 24, afin que cette dernière sorte toujours du manchon 4 dans une position déterminée et constante.

Le profil oblong interne 24 se termine par un chanfrein interne 26 incliné d'un angle A qui est dirigé en direction de l'extérieur du manchon 4 et permettant de positionner et de faciliter l'introduction de l'aiguille 2 à l'intérieur de l'alésage interne 5 du manchon 4 lorsqu'il est nécessaire de retirer ladite aiguille du site opératoire.

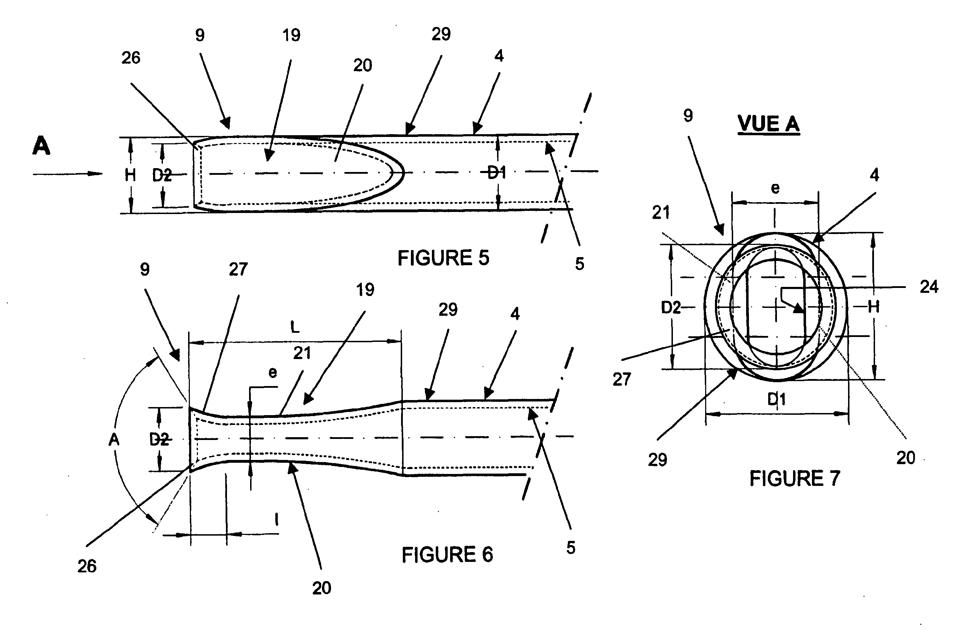

En figures 5 à 7 on a montré une variante du profil de l'extrémité libre 9 du manchon 4 lorsque ce dernier est constitué d'un tube métallique 29 autour duquel est surmoulé une enveloppe plastique cylindrique non représentée.

Les moyens d'indexation angulaire 19 présentent un profil semblable à celui décrit précédemment en figures 2 à 4, mais qui permettent une meilleur introduction de l'aiguille 2 à l'intérieur de l'alésage interne 5 pour son extraction du site opératoire.

On remarque que le profil interne oblong 24 communiquant avec l'alésage interne 5 du manchon 4 présente au niveau du chanfrein interne 26 un profil circulaire 27 de diamètre externe D2 qui est inférieur à celui D1 dudit manchon donnant à l'extrémité libre 9 une forme sensiblement de trompette.

Le profil circulaire 27 facilite l'introduction de l'aiguille 2 à l'intérieur de l'alésage interne 5 du manchon 4 lors de son extraction du site opératoire, car la surface d'introduction est supérieure à celle du profil oblong 24 décrit précédemment.

En figures 9 et 10 on a montré le manchon 4 qui est réalisé intégralement en plastique sans l'utilisation du tube métallique 29.

Dans ce cas le manchon 4 comporte un alésage interne 5 dont l'extrémité libre 9 présente des moyens d'indexation 19 obtenus lors du moulage dudit manchon.

Les moyens d'indexation angulaire 19 consistent en une conformation du diamètre interne de l'alésage 5 du manchon 4 lors de son moulage, afin de présenter un profil particulier qui permet lors de l'extraction de l'aiguille chirurgicale 2 de la diriger dans une position déterminée et constante par rapport aux axes principaux du manchon 4.

Ainsi, l'alésage interne 5 présente un profil interne oblong 24 semblable à celui décrit précédemment pour le tube métallique 29.

La conformation de l'alésage interne 5 permet de diriger l'aiguille 2 dans le sens du profil interne oblong 24, afin que cette dernière sorte toujours du manchon 4 dans une position déterminée et constante.

Le profil oblong interne 24 se termine par un chanfrein interne 26 qui est dirigé en direction de l'extérieur du manchon 4 et permettant de positionner et de faciliter l'introduction de l'aiguille 2 à l'intérieur de l'alésage interne 5 du manchon 4 lorsqu'il est nécessaire de retirer ladite aiguille du site opératoire.

Le manchon 4 comprend à partir de l'extrémité 9, et en direction de la tête 6, deux rainures 30 et 31 diamétralement opposées et parallèles à l'axe longitudinal de l'applicateur 1.

Chaque rainure 30 et 31 présente un profil en forme de V dont la base la plus ouverte, ou la plus large, est tournée du côté de l'extrémité 9. Egalement, chaque rainure 30 et 31 présente une profondeur décroissante le long de la périphérie du manchon 4, afin que le niveau le plus profond soit du côté de l'extrémité libre 9 et de la base la plus ouverte du V.

L'extrémité 9 du manchon 4 comporte une fente 32 disposée perpendiculairement aux rainures 30, 31 de manière que chaque rainure coopère avec ladite fente. Le diamètre de la fente 32 est sensiblement identique aux dimensions de la base la plus ouverte du V formant le profil de chaque rainure 30, 31.

La fente 32 forme une creusure à l'intérieur du manchon 4 qui communique avec l'alésage interne 5 dans lequel coulisse la tige 13 et l'aiguille 2 solidaire du fil de suture 3.

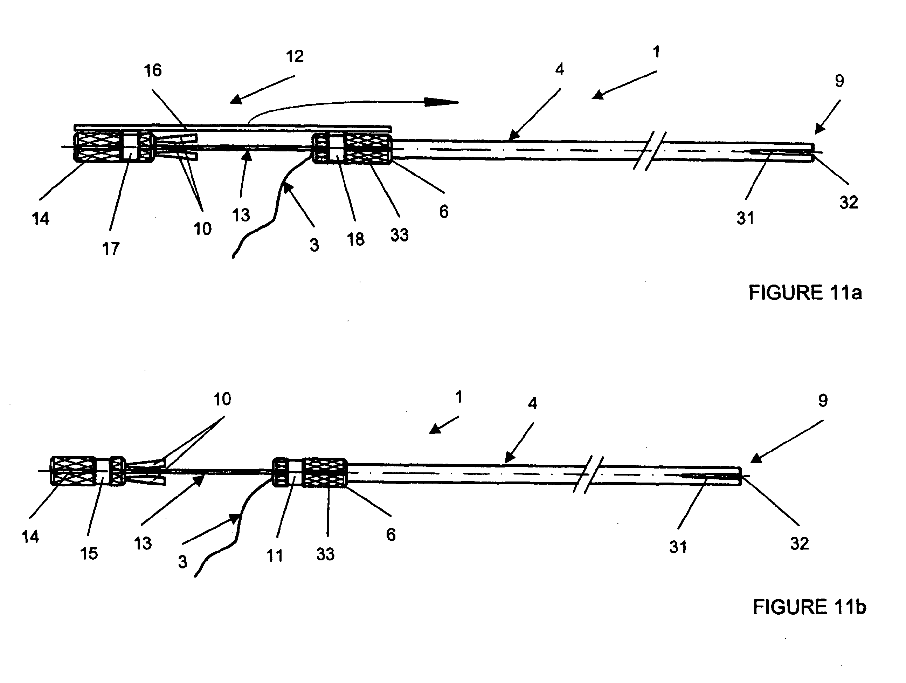

Enfin, la tête 6 du manchon 4 comporte respectivement dans l'alignement des rainures 30, 31 une empreinte 33 formant un repère au chirurgien pour positionner la fente 32, et les rainures dans le site opératoire (figures 11a à 11 h).

En effet, le manchon 4 muni des rainures 30, 31, de la fente 32 et des empreintes 33, permet au chirurgien de réaliser des noeuds sur le fil de suture 3 à l'extérieur du corps humain.

En figures 11a à 11h on a montré les différentes étapes pour l'utilisation de l'applicateur 1 suivant la présente invention.

L'applicateur 1 est prêt à être utilisé. Le chirurgien procède à son introduction dans le site opératoire (figure 11a).

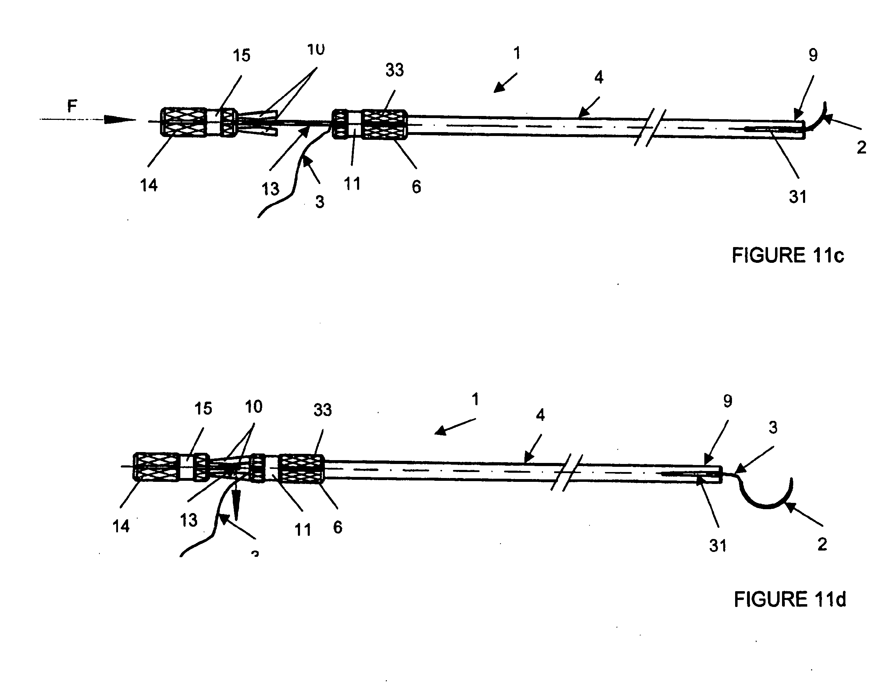

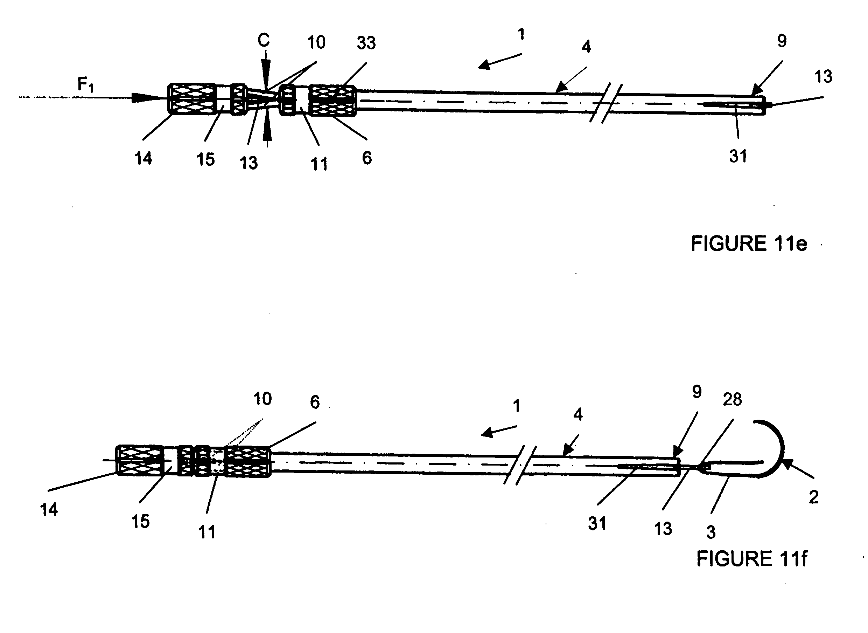

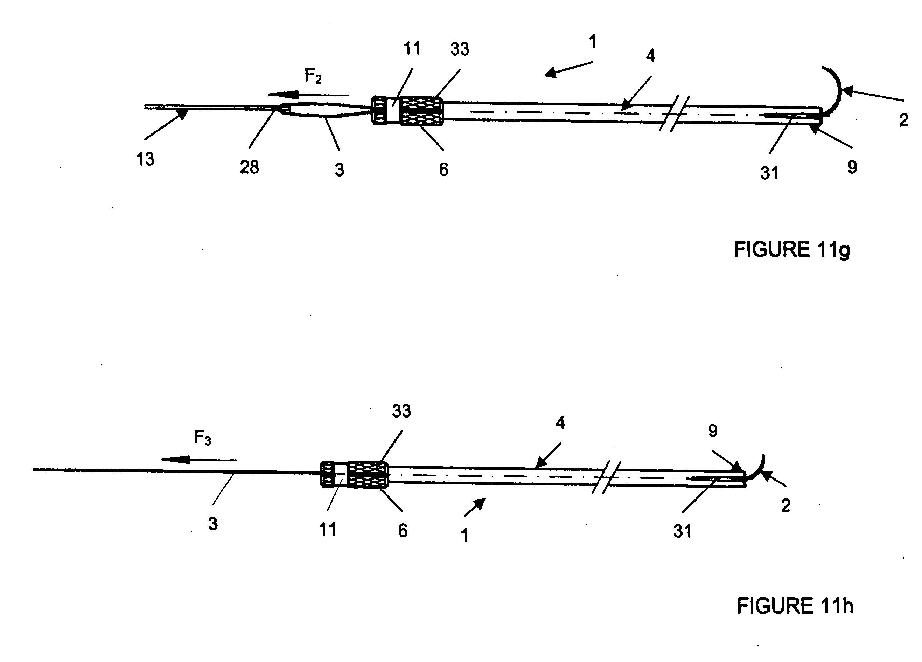

Dès que l'applicateur 1 est introduit par le chirurgien dans le site opératoire au travers d'un trocart non représenté, le chirurgien retire ensuite les moyens de retenue 12 pour libérer la tige 13 du manchon 4 (figure 11 b).

Le chirurgien appui sur la tête 14 de la tige 13 suivant la flèche F pour déplacer cette dernière à l'intérieur de l'alésage 5 afin de sortir l'aiguille 2 du manchon 4 dans une position déterminée en fonction de la position des moyens d'indéxation 19 (figure 11c). Dans cette position le chirurgien peut réaliser son premier point en utilisant le manchon 4 pour faire pénétrer l'aiguille 2 dans les tissus à recoudre.

En variante, le chirurgien peut continuer à pousser la tige 13 afin que les languettes 10 viennent en appui contre la tête 6 du manchon 4. Lorsque les languettes 10 sont en contact avec la tête 6 le chirurgien est certain que l'aiguille est complètement sortie du manchon 4 (figure 11d). Dans cette position le chirurgien récupère l'aiguille 2 à l'aide d'un porte aiguille pour réaliser son premier point et les suivants. Egalement, les languettes 10 en appui contre la tête 6 du manchon 4 empêchent l'extrémité libre de la tige 13 de pénétrer dans le site opératoire et de blesser les tissus avoisinant.

Pour récupérer l'aiguille 2 dans le site opératoire, le chirurgien comprime les languettes souples 10 suivant c, et pousse sur la tête 14 de la tige 13 suivant le flèche F1. La déformation des languettes 10 permet à ces dernières de pénétrer dans le logement 7 de la tête 6 et de prolonger le déplacement de la tige 13 à l'intérieur de l'alésage interne 5 du manchon 4 (figure 11e).

Ainsi, le chirurgien peut pousser sur la tête 14 de la tige 13 afin que cette dernière vienne en appui contre la tête 6 du manchon 4. Le contact entre les deux têtes 6 et 14 de l'applicateur 1 garanti au chirurgien que l'extrémité libre de la tige 13 pourvue du crochet 28 est à l'intérieur du site opératoire. Le chirurgien récupère à l'aide du crochet 28 le fil de suture 3 de l'aiguille 2 (figure 11f).

Le chirurgien tire ensuite la tige 13 suivant la flèche F2 pour l'extraire du manchon 4 et récupérer le fil de suture 3 au niveau de la tête 6. Dans cette position l'aiguille 2 est légèrement introduite dans le manchon 4 au niveau de son extrémité 9 (figure 11g).

Enfin, le chirurgien tire sur le fil de suture 3 suivant la flèche F3 pour introduire complètement l'aiguille 2 à l'intérieur du manchon 4 (figure 11 h).

L'applicateur 1 suivant la présente invention peut être utilisé pour réaliser des noeuds intra-corporel ou extracorporel suivant la technique de suture du chirurgien.

Il doit d'ailleurs être entendu que la description qui précède n'a été donnée qu'à titre d'exemple, et quelle ne limite nullement le domaine de l'invention dont on ne sortirait pas en remplaçant les détails d'exécution décrits par tout autre équivalent.

## Revendications

1. Applicateur pour la mise en place d'une aiguille chirurgicale (2) solidaire d'un fil de suture (3), comprenant un manchon (4) dans lequel est contraint dans une position sensiblement allongée l'aiguille chirurgicale (2), une tige (13) qui pénètre dans le manchon (4) pour l'extraction de ladite aiguille (2) et des moyens d'indexation angulaire (19) qui consistent en une conformation du manchon (4) au niveau de l'une de ses extrémités libres (9) de manière que l'alésage interne (5) dudit manchon (4) présente un profil interne oblong (24) dont les dimensions (e, H) sont différentes pour permettre lors de l'extraction de l'aiguille chirurgicale (2) du manchon (4) de la diriger dans une position déterminée et constante par rapport aux axes principaux dudit manchon.

2. Applicateur pour la mise en place d'une aiguille chirurgicale solidaire d'un fil de suture suivant la revendication 1, **caractérisé en ce que** les moyens d'indexation angulaire (19) consistent en une conformation du diamètre externe (D1) du manchon (4) afin que l'alésage interne (5) dudit manchon présente ledit profil interne oblong (24) dont la largeur (e) est inférieure au diamètre externe (D1), tandis que sa hauteur (H) est semblable à celle du diamètre externe (D1) pour permettre lors de l'extraction de l'aiguille chirurgicale (2) du manchon (4) de la diriger dans une position déterminée et constante par rapport aux axes principaux dudit manchon.

3. Applicateur pour la mise en place d'une aiguille chirurgicale solidaire d'un fil de suture suivant la revendication 2, **caractérisé en ce que** le diamètre extérieur (D1) du manchon (4) est déformé latéralement pour que l'extrémité (9) présente un profil qui est constitué de deux méplats parallèles (20, 21) se raccordant au profil cylindrique du manchon (4) par des portions inclinées (22, 23).

4. Applicateur pour la mise en place d'une aiguille chirurgicale solidaire d'un fil de suture suivant la revendication 3, **caractérisé en ce que** l'alésage interne (5) présente entre son profil interne oblong (24) et son diamètre circulaire interne une portion inclinée (25) provenant des portions inclinées extérieures (22, 23).

5. Applicateur pour la mise en place d'une aiguille chirurgicale solidaire d'un fil de suture suivant la revendication 2, **caractérisé en ce que** le profil oblong interne (24) se termine par une chanfrein interne (26) permettant de positionner et de faciliter l'introduction de l'aiguille (2) à l'intérieur de l'alésage interne (5) du manchon (4) lorsqu'il est nécessaire de retirer ladite aiguille du site opératoire.

6. Applicateur pour la mise en place d'une aiguille chirurgicale solidaire d'un fil de suture suivant la revendication 2, **caractérisé en ce que** le profil interne oblong (24) communiquant avec l'alésage interne (5) du manchon (4) présente au niveau du chanfrein interne (26) un profil circulaire (27) de diamètre externe (D2) qui est inférieur à celui (D1) dudit manchon donnant à l'extrémité libre (9) une forme sensiblement de trompette.

7. Applicateur pour la mise en place d'une aiguille chirurgicale solidaire d'un fil de suture suivant la revendication 2, **caractérisé en ce que** le profil du diamètre interne (5) du manchon (4) est obtenue par conformation d'un tube métallique (29).

8. Applicateur pour la mise en place d'une aiguille chirurgicale solidaire d'un fil de suture suivant la revendication 2, **caractérisé en ce que** le profil du diamètre interne (5) est obtenue par moulage du manchon (4).

9. Applicateur pour la mise en place d'une aiguille chirurgicale solidaire d'un fil de suture suivant la revendication 1, **caractérisé en ce que** la tige (13) comporte une tête (14) solidaire de languettes souples (10) permettant de limiter la course de la tige (13) par rapport au manchon (4).

10. Applicateur pour la mise en place d'une aiguille chirurgicale solidaire d'un fil de suture suivant la revendication 1, **caractérisé en ce qu'**il comprend des moyens de retenue (12) qui sont constitués d'une platine (16) de forme oblongue, dont l'une des faces est solidaire de deux pinces (17, 18) qui coopèrent respectivement avec la tige (13) et le manchon (4).

11. Applicateur pour la mise en place d'une aiguille chirurgicale solidaire d'un fil de suture suivant la revendication 10, **caractérisé en ce que** le manchon (4) comporte une tête cylindrique (6) pourvue sur sa face externe d'une empreinte circulaire (11) qui coopère avec la pince (18) de la platine (16), tandis que la tige (13) comprend une tête (14) munie d'une empreinte (15) autour de laquelle s'encliquette la pince (17) de ladite platine (16) de manière à maintenir une distance (L) constante entre les têtes (6, 14) pour assurer que l'extrémité libre de la tige (13) soit, d'une part en contact avec l'aiguille (2), et d'autre part à une distance (L') de l'extrémité libre (9) du manchon (4).

12. Applicateur pour la mise en place d'une aiguille chirurgicale solidaire d'un fil de suture suivant la revendication 1, **caractérisé en ce que** le manchon (4) comprend à partir de l'extrémité (9), et en direction de la tête (6), deux rainures (30, 31) diamétralement opposées et parallèles à l'axe longitudinal de l'applicateur.

13. Applicateur pour la mise en place d'une aiguille chirurgicale solidaire d'un fil de suture suivant la revendication 12, **caractérisé en ce que** chaque rainure (30, 31) présente un profil en forme de V dont la base la plus ouverte, ou la plus large, est tournée du côté de l'extrémité (9).

14. Applicateur pour la mise en place d'une aiguille chirurgicale solidaire d'un fil de suture suivant la revendication 13, **caractérisé en ce que** chaque rainure (30, 31) présente une profondeur décroissante le long de la périphérie du manchon (4), afin que le niveau le plus profond soit du côté de l'extrémité libre (9) et de la base la plus ouverte du V.

15. Applicateur pour la mise en place d'une aiguille chirurgicale solidaire d'un fil de suture suivant la revendication 12, **caractérisé en ce que** l'extrémité (9) du manchon (4) comporte une fente (32) disposée perpendiculairement aux rainures (30, 31) de manière que chaque rainure coopère avec ladite fente.

16. Applicateur pour la mise en place d'une aiguille chirurgicale solidaire d'un fil de suture suivant la revendication 15, **caractérisé en ce que** la fente (32) forme une creusure à l'intérieur du manchon (4) qui communique avec l'alésage interne (5).

## Claims

1. Applicator for positioning a surgical needle (2) which is integral with a suture thread (3), comprising a sleeve (4) in which the surgical needle (2) is stressed in a substantially elongate position, a rod (13) which penetrates into the sleeve (4) for extracting said needle (2), and angular indexing means (19) which consist of a shaping of the sleeve (4) at one of its free ends (9) such that the inner bore (5) of said sleeve (4) has an oblong inner profile (24) whose dimensions (e, H) are different so as to make it possible, during extraction of the surgical needle (2) from the sleeve (4), to direct said needle into a defined and constant position relative to the main axes of said sleeve.

2. Applicator for positioning a surgical needle which is integral with a suture thread according to Claim 1, **characterized in that** the angular indexing means (19) consist of a shaping of the external diameter (D1) of the sleeve (4) such that the inner bore (5) of said sleeve has said oblong inner profile (24) whose width (e) is less than the external diameter (D1), while its height (H) is similar to that of the external diameter (D1) so as to make it possible, during extraction of the surgical needle (2) from the sleeve (4), to direct said needle into a defined and constant position relative to the main axes of said sleeve.

3. Applicator for positioning a surgical needle which is integral with a suture thread according to Claim 2, **characterized in that** the external diameter (D1) of the sleeve (4) is deformed laterally so that the end (9) has a profile made up of two parallel flat parts (20, 21) connected to the cylindrical profile of the sleeve (4) via inclined portions (22, 23).

4. Applicator for positioning a surgical needle which is integral with a suture thread according to Claim 3, **characterized in that** the inner bore (5) has, between its oblong inner profile (24) and its internal circular diameter, an inclined portion (25) originating from the outer inclined portions (22, 23).

5. Applicator for positioning a surgical needle which is integral with a suture thread according to Claim 2, **characterized in that** the inner oblong profile (24) ends in an inner bevel (26) making it possible to position and more easily introduce the needle (2) inside the inner bore (5) of the sleeve (4) when it is necessary to retract said needle from the operating site.

6. Applicator for positioning a surgical needle which is integral with a suture thread according to Claim 2, **characterized in that** the oblong inner profile (24) communicating with the inner bore (5) of the sleeve (4) has, at the level of the inner bevel (26), a circular profile (27) of external diameter (D2) which is smaller than that (D1) of said sleeve, giving the free end (9) what is substantially a trumpet shape.

7. Applicator for positioning a surgical needle which is integral with a suture thread according to Claim 2, **characterized in that** the profile of the internal diameter (5) of the sleeve (4) is obtained by shaping a metal tube (29).

8. Applicator for positioning a surgical needle which is integral with a suture thread according to Claim 2, **characterized in that** the profile of the internal diameter (5) is obtained by molding the sleeve (4).

9. Applicator for positioning a surgical needle which is integral with a suture thread according to Claim 1, **characterized in that** the rod (13) comprises a head (14) integral with flexible tongues (10) making it possible to limit the travel of the rod (13) relative to the sleeve (4).

10. Applicator for positioning a surgical needle which is integral with a suture thread according to Claim 1, **characterized in that** it comprises retention means (12) which consist of a plate (16) of oblong shape, of which one of the faces is integral with two clamps (17, 18) which cooperate respectively with the rod (13) and the sleeve (4).

11. Applicator for positioning a surgical needle which is integral with a suture thread according to Claim 10, **characterized in that** the sleeve (4) comprises a cylindrical head (6) provided on its outer face with a circular impression (11) which cooperates with the clamp (18) of the plate (16), while the rod (13) comprises a head (14) equipped with an impression (15) about which the clamp (17) of said plate (16) clicks into place in such a way as to maintain a constant distance (L) between the heads (6, 14) so as to ensure that the free end of the rod (13) is on the one hand in contact with the needle (2) and on the other hand at a distance (L') from the free end (9) of the sleeve (4).

12. Applicator for positioning a surgical needle which is integral with a suture thread according to Claim 1, **characterized in that** the sleeve (4) comprises, starting from the end (9), and in the direction of the head (6), two grooves (30, 31) which are diametrically opposite one another and parallel to the longitudinal axis of the applicator.

13. Applicator for positioning a surgical needle which is integral with a suture thread according to Claim 12, **characterized in that** each groove (30, 31) has a V-shaped profile, of which the most open base, or widest base, is directed toward the end (9).

14. Applicator for positioning a surgical needle which is integral with a suture thread according to Claim 13, **characterized in that** each groove (30, 31) has a depth decreasing along the periphery of the sleeve (4) so that the deepest level is toward the free end (9) and the most open base of the V.

15. Applicator for positioning a surgical needle which is integral with a suture thread according to Claim 12, **characterized in that** the end (9) of the sleeve (4) comprises a slit (32) arranged perpendicular to the grooves (30, 31) in such a way that each groove cooperates with said slit.

16. Applicator for positioning a surgical needle which is integral with a suture thread according to Claim 15, **characterized in that** the slit (32) forms a hollow inside the sleeve (4) which communicates with the inner bore (5).

## Patentansprüche

1. Applikator zum Einbringen einer chirurgischen Nadel (2), die fest mit einem Nahtfaden (3) verbunden ist, umfassend eine Hülse (4), in der die chirurgische Nadel (2) in einer im Wesentlichen gestreckten Position eingezwängt ist, einen Stab (13), der zum Herausziehen der Nadel (2) in die Hülse (4) eindringt, und Mittel zur Winkelverstellung (19), die aus einer Form der Hülse (4) im Bereich eines ihrer freien Enden (9) bestehen, damit die Innenbohrung (5) der Hülse (4) ein längliches Innenprofil (24) aufweist, dessen Abmessungen (e, H) unterschiedlich sind, damit die chirurgische Nadel (2) beim Herausziehen aus der Hülse (4) in eine bestimmte Position gebracht werden kann, die im Verhältnis zu den Hauptachsen der Hülse gleichbleibend ist.

2. Applikator zum Einbringen einer chirurgischen Nadel, die fest mit einem Nahtfaden verbunden ist, nach Anspruch 1, **dadurch gekennzeichnet, dass** die Mittel zur Winkelverstellung (19) aus einer Form des Außendurchmessers (D1) der Hülse (4) bestehen, damit die Innenbohrung (5) der Hülse das längliche Innenprofil (24) aufweist, dessen Breite (e) kleiner als der Außendurchmesser (D1) ist, während seine Höhe (H) der des Außendurchmessers (D1) ähnelt, damit die chirurgische Nadel (2) beim Herausziehen aus der Hülse (4) in eine bestimmte Position gebracht werden kann, die im Verhältnis zu den Hauptachsen der Hülse gleichbleibend ist.

3. Applikator zum Einbringen einer chirurgischen Nadel, die fest mit einem Nahtfaden verbunden ist, nach Anspruch 2, **dadurch gekennzeichnet, dass** der Außendurchmesser (D1) der Hülse (4) seitlich verformt ist, damit das Ende (9) ein Profil aufweist, das aus den zwei parallelen Abflachungen (20, 21) besteht, die über die geneigten Abschnitte (22, 23) mit dem walzenförmigen Profil der Hülse (4) verbunden sind.

4. Applikator zum Einbringen einer chirurgischen Nadel, die fest mit einem Nahtfaden verbunden ist, nach Anspruch 3, **dadurch gekennzeichnet, dass** die Innenbohrung (5) zwischen ihrem länglichen Innenprofil (24) und ihrem kreisförmigen Innendurchmesser einen geneigten Abschnitt (25) aufweist, der von den geneigten Außenbereichen (22, 23) herrührt.

5. Applikator zum Einbringen einer chirurgischen Nadel, die fest mit einem Nahtfaden verbunden ist, nach Anspruch 2, **dadurch gekennzeichnet, dass** das längliche Innenprofil (24) in einer inneren Abschrägung (26) endet, durch die die Nadel (2) im Inneren der Innenbohrung (5) der Hülse (4) angeordnet und leichter eingeführt werden kann, wenn die Nadel aus der Operationsstelle herausgezogen werden muss.

6. Applikator zum Einbringen einer chirurgischen Nadel, die fest mit einem Nahtfaden verbunden ist, nach Anspruch 2, **dadurch gekennzeichnet, dass** das längliche Innenprofil (24), das mit der Innenbohrung (5) der Hülse (4) in Verbindung steht, im Bereich der inneren Abschrägung (26) ein kreisförmiges Profil (27) mit dem Außendurchmesser (D2) aufweist, der kleiner als der (D1) der Hülse ist, wodurch dem freien Ende (9) im Wesentlichen die Form einer Trompete verliehen wird.

7. Applikator zum Einbringen einer chirurgischen Nadel, die fest mit einem Nahtfaden verbunden ist, nach Anspruch 2, **dadurch gekennzeichnet, dass** das Profil des Innendurchmessers (5) der Hülse (4) durch die Formung eines Metallrohrs (29) erhalten wird.

8. Applikator zum Einbringen einer chirurgischen Nadel, die fest mit einem Nahtfaden verbunden ist, nach Anspruch 2, **dadurch gekennzeichnet, dass** das Profil des Innendurchmessers (5) durch die Formung der Hülse (4) erhalten wird.

9. Applikator zum Einbringen einer chirurgischen Nadel, die fest mit einem Nahtfaden verbunden ist, nach Anspruch 1, **dadurch gekennzeichnet, dass** der Stab (13) einen Kopf (14) umfasst, der fest mit den flexiblen Streifen (10) verbunden ist, mit denen die Bewegung des Stabs (13) zur Hülse (4) eingeschränkt werden kann.

10. Applikator zum Einbringen einer chirurgischen Nadel, die fest mit einem Nahtfaden verbunden ist, nach Anspruch 1, **dadurch gekennzeichnet, dass** er Rückhaltemittel (12) umfasst, die aus einer Platte (16) mit länglicher Form bestehen, von der eine Seite fest mit den beiden Klemmen (17, 18) verbunden ist, die mit dem Stab (13) beziehungsweise der Hülse (4) zusammenwirken.

11. Applikator zum Einbringen einer chirurgischen Nadel, die fest mit einem Nahtfaden verbunden ist, nach Anspruch 10, **dadurch gekennzeichnet, dass** die Hülse (4) einen walzenförmigen Kopf (6) umfasst, der auf seiner Außenseite mit einer kreisförmigen Vertiefung (11) versehen ist, die mit der Klemme (18) der Platte (16) zusammenwirkt, während der Stab (13) einen Kopf (14) umfasst, der mit einer Vertiefung (15) versehen ist, um die herum die Klemme (17) der Platte (16) einrastet, damit ein gleich bleibender Abstand (L) zwischen den Köpfen (6, 14) aufrechterhalten wird, um sicherzustellen, dass das freie Ende des Stabs (13) einerseits die Nadel (2) berührt und andererseits einen Abstand (L') zum freien Ende (9) der Hülse (4) aufweist.

12. Applikator zum Einbringen einer chirurgischen Nadel, die fest mit einem Nahtfaden verbunden ist, nach Anspruch 1, **dadurch gekennzeichnet, dass** die Hülse (4) vom Ende (9) ausgehend und in Richtung des Kopfs (6) die beiden Nuten (30, 31) aufweist, die einander diametral entgegengesetzt sind und parallel zur Längsachse des Applikators verlaufen.

13. Applikator zum Einbringen einer chirurgischen Nadel, die fest mit einem Nahtfaden verbunden ist, nach Anspruch 12, **dadurch gekennzeichnet, dass** jede Nut (30, 31) ein Profil in Form eines V aufweist, dessen am weitesten offene oder breiteste Basis zum Ende (9) gedreht ist.

14. Applikator zum Einbringen einer chirurgischen Nadel, die fest mit einem Nahtfaden verbunden ist, nach Anspruch 13, **dadurch gekennzeichnet, dass** jede Nut (30, 31) entlang des Rands der Hülse (4) eine abnehmende Tiefe aufweist, sodass der tiefste Bereich beim freien Ende (9) und der am weitesten offenen Basis des V liegt.

15. Applikator zum Einbringen einer chirurgischen Nadel, die fest mit einem Nahtfaden verbunden ist, nach Anspruch 12, **dadurch gekennzeichnet, dass** das Ende (9) der Hülse (4) einen Schlitz (32) aufweist, der senkrecht zu den Nuten (30, 31) angeordnet ist, damit jede Nut mit dem Schlitz zusammenwirkt.

16. Applikator zum Einbringen einer chirurgischen Nadel, die fest mit einem Nahtfaden verbunden ist, nach Anspruch 15, **dadurch gekennzeichnet, dass** der Schlitz (32) im Inneren der Hülse (4) eine Vertiefung bildet, die mit der Innenbohrung (5) in Verbindung steht.
